# EUROPEAN PATENT APPLICATION

(11) **EP 1 564 300 A1**
(43) Date of publication of application: **17.08.2005**
(21) Application number: 02781745.1
(22) Date of filing: 13.11.2002
(51) Int. Cl.: C12Q 1/68, C12N 15/09, C12M 1/00

(54) **HETHOD OF AMPLIFYING NUCLEIC ACID AND APPARATUS THEREFOR**

(71) Applicant: Hitachi High-Technologies Corporation, Tokyo 105-8717 (JP)
(72) Inventor: KANDA, T., Design&Manufacturing Gr., Naka Div., Hitachinaka-shi, Ibaraki 312-8504 (JP); FUKUZONO, S., Design&Manufacturing Gr., Naka Div., Hitachinaka-shi, Ibaraki 312-8504 (JP); TAKAHASHI, S., Design&Manufacturing Gr., Naka Div., Hitachinaka-shi, Ibaraki 312-8504 (JP)
(74) Representative: Strehl Schübel-Hopf & Partner
(86) International application number: PCT/JP2002/011815
(87) International publication number: WO 2004/044243

(57) **Abstract**

In nucleic acid amplification, the amount of a labeled or modified oligomer used is reduced to lower cost, and detection is enabled within the measurement range of a detection unit without requiring operations such as dilution. A nucleic acid measurement method of the invention comprises the steps of extracting a target nucleic acid, mixing an oligomer labeled with a luminescent substance or a modification group with another oligomer that has the same base sequence with that of the labeled oligomer and that is not labeled with a luminescent substance or a modification group, amplifying the target nucleic acid using the mixture of the labeled oligomer and the unlabeled oligomer, adding a luminescent substance to an oligomer labeled with a modification group and its amplification product, and measuring the amplified target nucleic acid using a photodetector.

SEQUENCE LISTING

<110> HITACHI HIGH-TECHNOLOGIES CORPORATION

<120> METHOD AND APPARATUS FOR DETECTING NUCLEIC ACID

<130> PH-1502-PCT

<140>
<141>

<160> 3

<170> PatentIn Ver. 2.1

<210> 1
<211> 20
<212> DNA
<213> Artificial Sequence

<400> 1
GGGCTTGACT TTCCAACACG 20

<210> 2
<211> 20
<212> DNA
<213> Artificial Sequence

<400> 2

GGGCTTGACT TTCCAACACG 20

<210> 3
<211> 50
<212> DNA
<213> Artificial Sequence

<400> 3
TCTAGCCTCA ATCCTCATAC 20

## Description

### TECHNICAL FIELD

The present invention relates to a method of amplifying target nucleic acids, reagents for detection thereof, and an apparatus therefor, which are to be used for diagnosis and research purposes in the fields of biochemistry, molecular biology and medicine, among others.

### BACKGROUND ART

As the technology of detecting trace amounts of nucleic acids, the detection of nucleic acids formed by various methods of nucleic acid amplification using a labeled oligomer(s) or terminator(s) is in current use.

For example, the PCR (polymerase chain reaction) method comprises synthesizing two oligomers complementary in sequence to portions of the target single- or double-stranded nucleic acid and adding them in excess to a reaction mixture in the presence of a thermostable DNA polymerase. If the target nucleic acid is present in the reaction mixture, the oligomers bind to specific sites of the respective strands thereof. And, the polymerase causes successive addition of nucleotides complementary to the target nucleic acid strands for extension of the 3' terminal of each oligomer. When the temperature is raised and lowered continuously, the extended oligomers separate as product nucleic acids from the target nucleic acid strands and, like the original target nucleic acid strands, can bind to the oligomers. By repeating this process, it is possible to exponentially amplify the target nucleic acid between the two oligomers.

The LCR (ligase chain reaction) method comprises adding two neighboring oligonucleotide probes and probes complementary thereto in excess to a reaction mixture in the presence of a heat-stable DNA ligase. Each probe then binds to the target sequence, and the thus-bound two neighboring probes are ligated together by means of the DNA ligase. Like in the PCR method, when the temperature is raised and lowered continuously, the probes ligated together leave the target sequence and are bound to the probes, like the original target nucleic acid.

Further, the RCR (repair chain reaction) method, TAS method, 3SR method, NASBA method, SPSR method, LAT method, SDA method and like methods are in use as other methods of nucleic acid amplification.

Among these, the PCR method can be used as a high-sensitivity method of analyzing nucleic acids in various samples and is utilized in diagnosing infectious diseases, hereditary diseases and cancer, among others. Furthermore, the PCR method is a method also suited for use in DNA typing tests on the occasion of transplantation or parentage diagnosis. In that case, the nucleic acids to be analyzed or tested are often subjected to the amplification reaction using labeled oligomers. At present, even when they are present only in very small amounts, they can be detected owing to the development of chemical substances for use as labels and the improvement in detection apparatus.

For the above-mentioned method of nucleic acid amplification, various kits have been marketed and the method of labeling has been simplified and, generally, the target nucleic acids are abundantly amplified using luminescent substance-labeled oligomers. In detecting the thus-amplified target nucleic acids on the current sequencers or plate readers improved in detection sensitivity, it is a general practice to dilute the samples so that the target nucleotide concentrations may fall within the detection range of the apparatus to be used. For that purpose, a complicated procedure including sample distribution and dilution becomes necessary to optimize the concentration of the product nucleic acid for enabling measurement thereof on the detection apparatus. In cases where the amplification of the target nucleic acid is automated, a step and apparatus parts for dilution, for instance, are required.

Furthermore, the above-mentioned methods of measurement use only one of dozens of parts of the nucleic acid formed in each measurement. Further, the number of measurements of the nucleic acid formed is several in most cases. Therefore, it may be said that unnecessarily labeled oligomers are used. However, the current methods of nucleic acid amplification require the use of oligomers in amounts not smaller than certain levels relative to the target template nucleic acid and, therefore, it is impossible to simply reduce the amount of labeled oligomers. With the advancement in gene diagnosis and the like in the future, it will become necessary to amplify the same target site in a number of samples. If the amounts of labeled oligomers to be used can be reduced in such a case, it will become possible to reduce the cost necessary for each sample.

To solve these problems, the advent of a method of amplifying labeled nucleic acids which can be carried out in a simple and easy manner and at low cost is waited for.

### DISCLOSURE OF THE INVENTION

In accordance with the present invention, arbitrary nucleic acids are amplified using a mixture of a labeled or modified oligomer and an oligomer labeled or unlabeled with the same label. The mixture is prepared so that the amount of the label or luminescent substance in the solution containing the product nucleic acid may fall within the measurement range of the detection apparatus. When a modified oligomer is used, a luminescent substance is bound to the modifying group.

According to the present invention, the amount of the labeled or modified oligomer can be reduced as compared with the prior art methods and, therefore, the cost for amplification can be reduced. The procedure for diluting the nucleic acid formed can be omitted, so that the operation time can be shortened particularly when one and the same target nucleic acid is to be amplified in a large number of samples.

In the following, the novel characteristic features and effects of the present invention in the above-mentioned and other aspects are described referring to the drawings attached hereto. Several terms used herein are defined as follows.

The term "label" includes, within the meaning thereof, a fluorescent substance, a luminescent substance and a radioisotope as added to an oligomer for detecting the nucleic acid formed.

The "labeling or labeled" refers to the addition of the fluorescent substance, luminescent substance or radioisotope to an oligomer or nucleic acid.

The "target nucleic acid" includes the nucleic acid comprising a base sequence to be arbitrarily amplified among the nucleic acids contained in the reaction mixture in the amplification reaction.

The "product nucleic acid" and "product DNA" include the nucleic acid and DNA formed in the amplification reaction, irrespective of their being labeled or modified or not.

The "sequence related to the target nucleic acid" includes a part or the whole of a sequence identical or complementary to the base sequence of the target nucleic acid.

The "template nucleic acid" includes any nucleic acid having the target nucleic acid and occurring at the start of the reaction. Although the product nucleic acid also serves as the template in the amplification reaction, that nucleic acid is distinguished herein from the template nucleic acid.

### BRIEF DESCRIPTION OF DRAWINGS

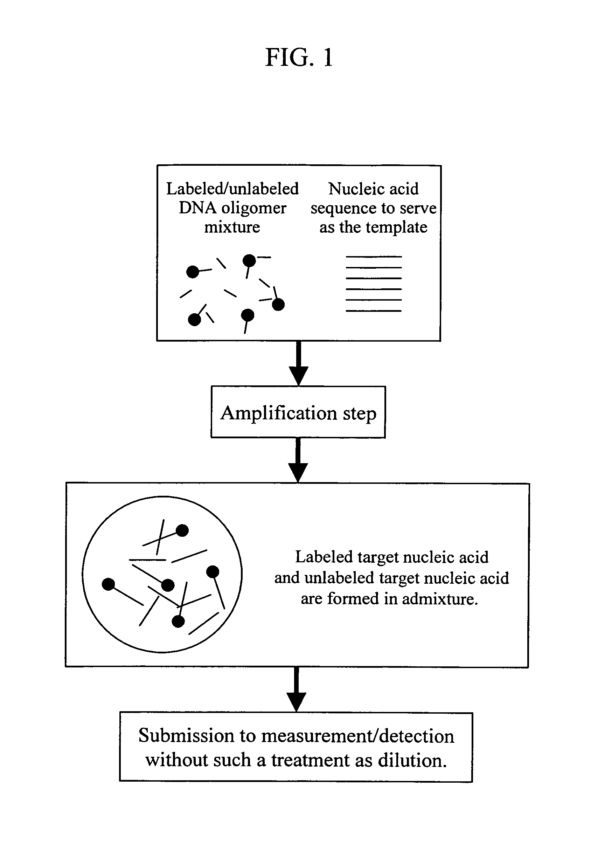

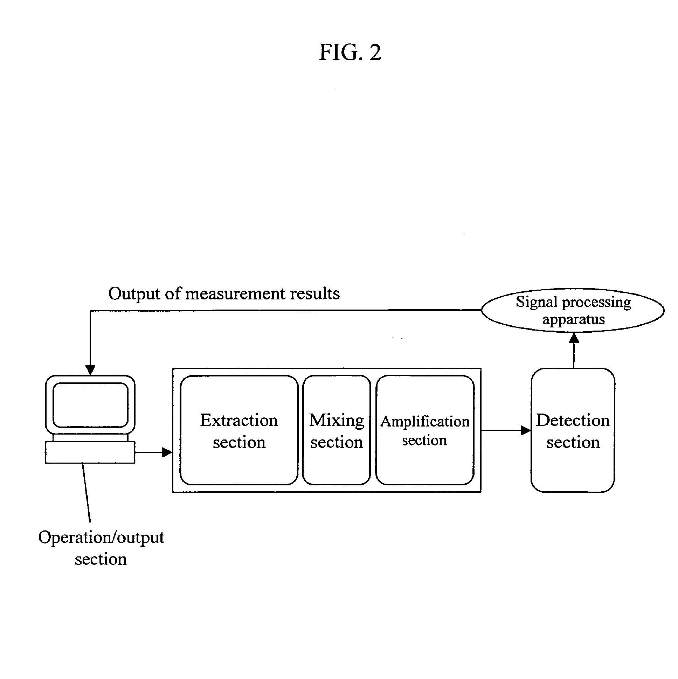

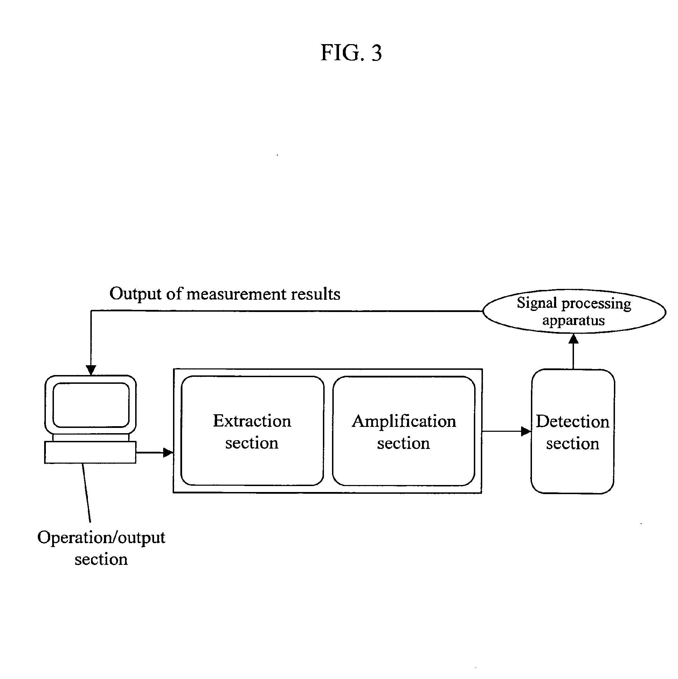

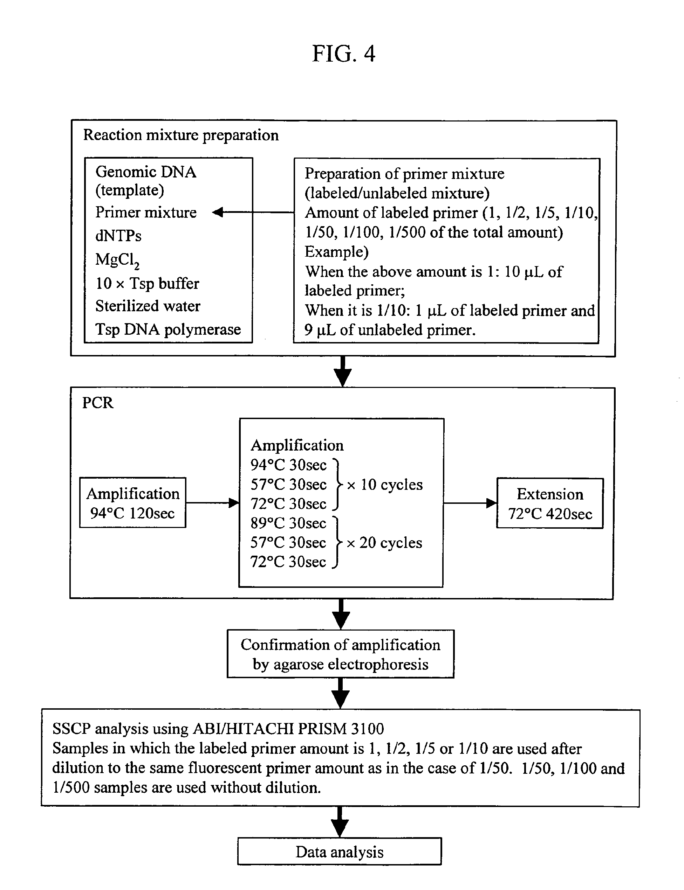

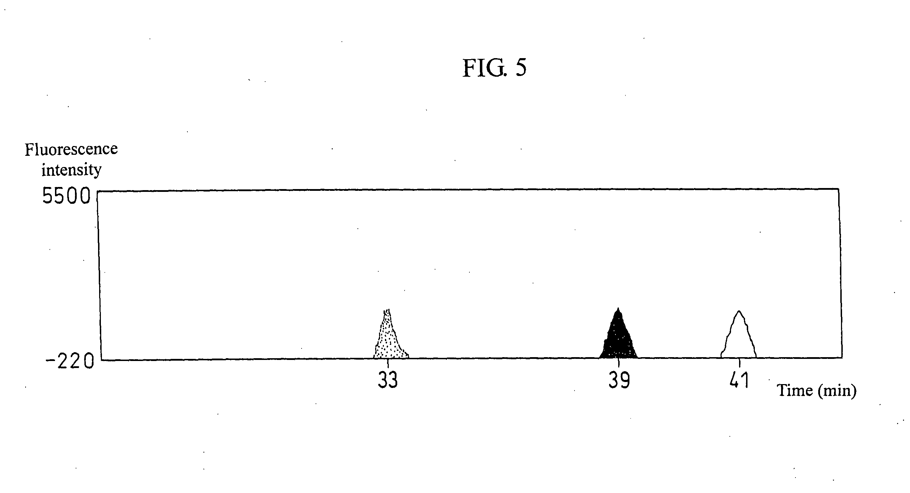

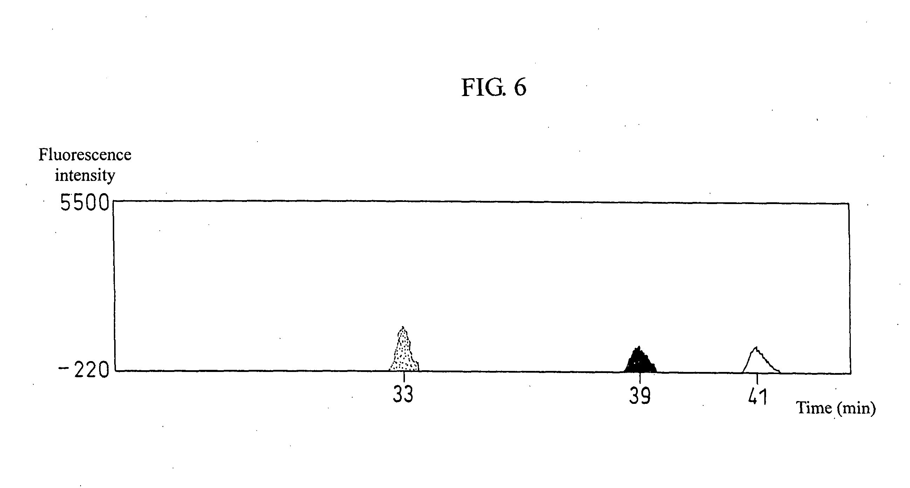

Fig. 1 is a schematic representation of a method of forming labeled nucleic acids. Fig. 2 is an example of the nucleic acid analyzing apparatus to which the present invention is applied. Fig. 3 is a variation of the nucleic acid analyzing apparatus shown in Fig. 2. Fig. 4 is a schematic representation of the nucleic acid amplification method described in Example 1. Fig. 5 shows the results of analysis as obtained at a proportion of the labeled ALD-F primer of 1/50. Fig. 6 shows the results of analysis as obtained at a proportion of the labeled ALD-F primer of 1/100.

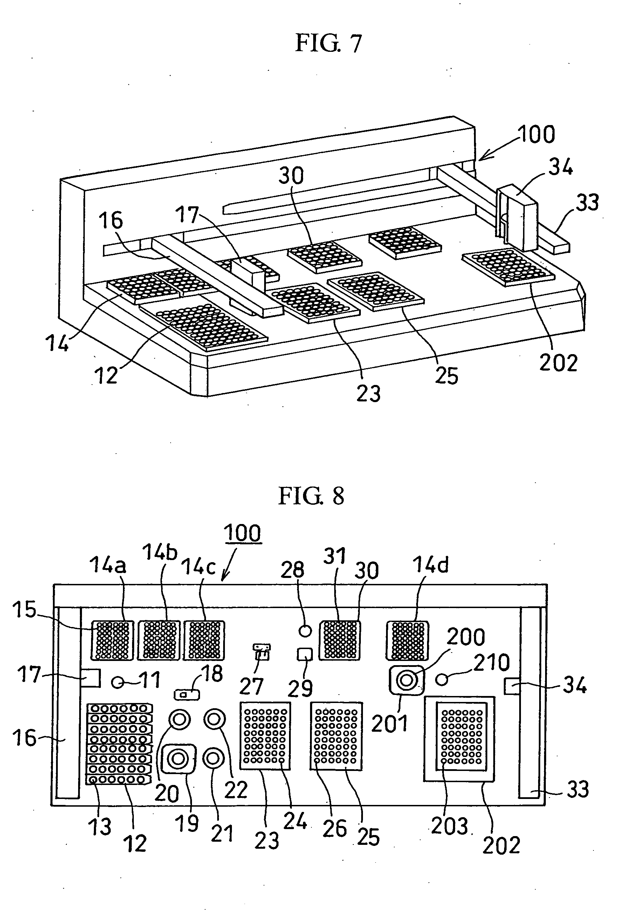

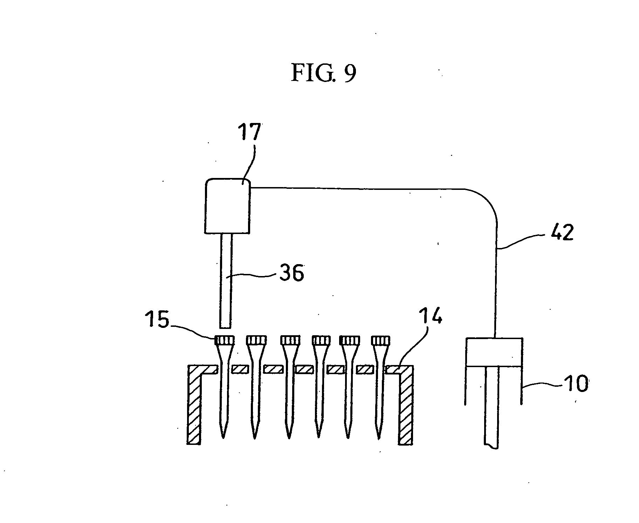

Fig. 7 is an external view of the extraction/amplification part 100 used in Example 2, Fig. 8 is a plan view of the extraction/amplification part 100 used in Example 2, and Fig. 9 is an illustration of the operation for attaching tips to the nozzle in Example 2.

### BEST MODES FOR CARRYING OUT THE INVENTION

The nucleic acid amplification method, for example the PCR method, is a method comprising selecting two sites sandwiching the target segment of a single-stranded or double-stranded DNA, allowing oligomers serving as origins of replication to attach to the two sites, and carrying out the reaction using DNA polymerase to amplify the required segment alone.

The oligomers used on that occasion each generally comprises 20 to 25 bases and, when a label is required at the site to be amplified, a labeling substance, such as a luminescent substance, is coupled to the oligomer(s).

According to the prior art methods of labeling, the labeled oligomer alone is added to the reaction mixture to a concentration thereof of 0.2 µM to 2 µM and, after amplification, the mixture is diluted so that the concentration may fall within the measurement range of the detection apparatus and, then, measurement/detection is carried out.

In accordance with the present invention, the amplification reaction (e.g. PCR, LCR) of a target nucleic acid is carried out using a labeled oligomer (hereinafter referred to as "oligomer A") and an unlabeled oligomer (hereinafter, "oligomer B") identical in base sequence to the oligomer mentioned above, as illustrated in Fig. 1. On that occasion, the oligomer A and oligomer B may not be identical in base sequence but may have arbitrary specific sequences contained in the target nucleic acid. The oligomer A and oligomer B may be different in the number of bases.

These two oligomers are mixed together in a mixing ratio of 1:1 to 1:10,000, more preferably 1:10 to 1:500, and the amplification reaction is carried out at a total concentration of both in the reaction mixture within the range of 0.2 µM to 2 µM. After the amplification step, a mixture of a labeled nucleic acid and an unlabeled nucleic acid is found formed and this can be subjected to measurement/detection, for instance, without such concentration adjustment as dilution.

When the target nucleic acid amplification, dilution of the product nucleic acid, detection and analysis according to the prior art are compared with the amplification, detection and analysis according to the present invention, both can give the same results.

Fig. 2 schematically shows a nucleic acid detecting and analyzing apparatus according to the invention. This apparatus is constituted of an extraction section, mixing section, amplification section, detecting section, signal processing apparatus and operation/output section. The extraction section comprises a mechanism for extracting the template nucleic acid from a sample of a living organism. For example, the apparatus described in JP-A 2000-166556 is utilized. The mixing section comprises a mechanism for preparing an oligomer mixture by mixing up the oligomer A and oligomer B in specified amounts. The amplification section comprises a mechanism for mixing up the nucleic acid, oligomer mixture, and nucleic acid amplification reagents and amplifying the desired specific nucleic acid by the PCR method or constant-temperature amplification method, for instance. The detection section and signal processing apparatus are mechanisms for detecting the specific nucleic acid amplified. The operation/output section can control the respective mechanisms mentioned above, and detect and analyze the specific nucleic acid contained in the living organism-derived sample. There may also exist such a variation as a nucleic acid detecting/analyzing apparatus utilizing an oligomer mixture already prepared and which has no mixing section, as shown in Fig. 3.

The detection and analysis are carried out in the following manner. First, the detecting/analyzing procedure is carried out through the operation/output section. According to this order, the nucleic acid to serve as the template is extracted in the extraction section. Then, in the mixing section, a labeled or modifying group-coupled oligomer is mixed with an unlabeled or modifying group-free oligomer identical in base sequence to the labeled or modifying group-coupled oligomer. The target nucleic acid is amplified in the amplification section using this oligomer mixture. In the detecting section, the target nucleic acid amplified is detected or analyzed. The detection data are processed in the signal processing apparatus, and the results are sent to the output section. For efficient detection/analysis purposes, it is preferable to constitute an apparatus in which these sections are integrated. In this manner, in the practice of the invention, it is not always necessary to provide a mechanism for diluting the target nucleic acid amplified between the amplification and detecting sections.

The reaction mixture for amplifying the target nucleic acid is prepared using reagents in very common use, namely a buffer solution (e.g. Tris-hydrochloride buffer), a thermostable DNA polymerase (e.g. Taq DNA polymerase), a salt (e.g. magnesium chloride) and deoxyribonucleotide triphosphates, as well as the template DNA (e.g. human genomic DNA). The reaction is carried out in the conventional manner in temperature cycles involving denaturation, annealing, and extension but the conditions may vary to some extent according to the sequences and numbers of bases of the target nucleic acid and DNA oligomers.

In cases where a PCR kit (e.g. Takara Shuzo's PCR Amplification Kit) for general use which does not include oligomers for amplification is used, the oligomer A and oligomer B are added to the reaction mixture in a mixing ratio of 1:1 to 1:10,000, more preferably 1:10 to 1:500 and to the same total oligomer concentration as specified for the kit, and the reaction is carried out according to the temperature cycle instructed.

In cases where another reaction other than the amplification of the target nucleic acid is simultaneously carried out (e.g. in RT-PCR), too, the oligomer A and oligomer B are mixed together in a mixing ratio of 1:1 to 1:10,000, more preferably 1:10 to 1:500, and the reaction is carried out using the mixture in a total amount giving a concentration suited for the reaction. In this manner, the luminescence intensity or radioactivity in the product nucleic acid becomes adequate for the detection or analysis.

As the nucleic acid amplification method, there may be mentioned not only the above-mentioned PCR method but also the RCR (repair chain reaction), TAS, 3SR, NASBA, SPSR, LAT, and SDA methods. Any of such and other methods that are carried out using the above-mentioned oligomer mixture may be employed without any particular restriction.

After completion of the amplification reaction, detection or analysis is carried out on the detection apparatus without diluting. On that occasion, when the detection apparatus is not one detecting the product nucleic acid while isolating the same (e.g. DNA sequencer) but one detecting the product nucleic acid in the reaction mixture (e.g. fluorescence plate reader), the unreacted oligomers are removed.

When the amplification reaction is carried out using a modifying group-coupled oligomer, a treatment for coupling of a luminescent substance, which can be detected or measured by an apparatus equipped with a photodetector such as a photoelectron multiplier, photodiode, or CCD, via the modifying group is carried out after the amplification reaction and then the measurement or detection is carried out. In the nucleic acid detection and analyzing apparatus of the invention, the treatment for coupling of a luminescent substance via the modifying group is carried out in the amplification section after completion of the amplification reaction and, thereafter, the detection or analysis is carried out.

The substance for labeling the oligomer to be used in the amplification reaction is not particularly restricted but may be any substance that can be detected on an apparatus provided with a detecting section in accordance with the invention in the step of detecting the target nucleic acid. Thus, it may be a fluorescent substance, chemiluminescent substance, or radioisotope. These are high in luminescence intensity, and very small amounts thereof can be detected or analyzed. Useful as the modifying group are the phosphoric acid group, amino group, thiol group, biotin, and digoxigenin, among others. A fluorescent substance or chemiluminescent substance can be readily coupled to any of these modifying groups.

The method of detecting the target nucleic acid amplified is not particularly restricted but may be any method that can detect the same on the above-mentioned detector. Thus, the measurement apparatus includes, but is not particularly restricted to, apparatuses equipped with such a detecting section as a DNA analyzer, gel imaging analyzer, fluorophotometer, sequencer, or plate reader.

In nucleic acid amplification according to the prior art methods, all product nucleic acid molecules are labeled or have a modifying group coupled thereto and, accordingly, the labeled or modified target nucleic acid is prepared in large excess. For optimizing the concentration of the product nucleic acid so that measurement may become possible on the detection apparatus, a complicated procedure comprising distributing the sample and diluting the same, for instance, is required. For automatic amplification of the target nucleic acid, for instance, the step and apparatus parts for dilution are required, among others. In accordance with the present invention, the amount of the fluorescent substance, luminescent substance or radioisotope to be used for labeling or modification can be reduced and, therefore, cost reduction becomes possible. Further, the nucleic acid treatment steps can be simplified.

The following examples illustrate the present invention more specifically. They are, however, by no means limitative of the scope of the invention.

### Example 1

A method of detecting a specific gene (e.g. aldolase gene as a target sequence) region is described in this example. This method comprises the step of extracting the template DNA from a sample, the step of preparing a primer set for amplifying the target nucleic acid segment in the template DNA, the amplification reaction step, and the detection step.

### [Step of extracting the template DNA from a sample]

Usable as the sample are human peripheral blood (whole blood), tissue sections obtained by biopsy, and urine, for instance. The genomic DNA is extracted from these by a method well known in the art. In the case of human peripheral blood, for instance, Qiagen's DNA Blood Mini Kit can be used. Specifically, the standard protocol attached to the kit is followed. Thus, 200 µL of human whole blood and 20 µL of Qiagen Protease are added to a 1.5-ml microtube. Then, 200 µL of Buffer AL is added and, after thorough mixing using a vortex mixer, incubation is carried out at 56°C for 10 minutes. Then, the microtube is subjected to centrifugation, the liquid is collected, 200 µL of ethanol is added, and the mixture is thoroughly stirred using again a vortex mixer. The whole mixture is poured into a spin column in a collection tube and centrifuged at 8,000 rpm for 1 minute to allow the liquid to pass through the column. The genomic DNA is trapped within the column. Then, the same spin column is transferred to a new collection tube, the cover is removed, 500 µL of Buffer A W 1 is poured into the tube, and centrifugation is carried out at 8,000 rpm for 1 minute. The spin column is again transferred to a new collection tube, 500 µL of Buffer AW2 is then poured into the tube, and the column is washed by 3 minutes of centrifugation at 14,000 rpm. The spin column is transferred to a new collection tube, 100 µL of Buffer AE is poured into the tube, and the genomic DNA trapped is eluted by 1 minute of centrifugation at 8,000 rpm to give a genomic DNA solution. The amount of the nucleic acid in the eluate is determined by measuring the eluate genomic DNA solution for absorbance using an absorptiometer.

In the case of urine, the urine collected in a centrifuge tube is centrifuged at 1,000 rpm for 5 minutes, the sediment alone is recovered, physiological saline is added to the sediment for redispersion and washing, centrifugation is again carried out at 1,000 rpm for 5 minutes, and the sediment is recovered for use as the sample. Then, the sample is subjected to proteinase K digestion, which is well known in the art, and the genomic DNA is extracted by phenol-chloroform extraction, for instance. Tissue sections can be treated in the same manner. A urine sediment, tissue section or blood-derived leucocytes frozen stored at -80°C may also be used as the sample.

### [Step of preparing a primer set for amplifying the target nucleic acid segment]

The following three polynucleotides were prepared as the primers for PCR amplification.

5'FAM-GGGCTTGACTTTCCAACACG3'  (ALD-FF primer)

5'GGGCTTGACTTTCCAACACG3' (ALD-FN primer)

5'TCTAGCCTCAATCCTCATAC3'          (ALD-R primer)

Both the ALD-FF primer and ALD-FN primer are polynucleotides having the same sequence. They hybridize with a portion of the aldolase gene region, which is the target sequence, and both are used as forward primers in the PCR amplification reaction. The ALD-FF primer is a labeled polynucleotide having FAM, which is a fluorescent substance, bound thereto at the 5' terminus thereof and can be detected by fluorescence detection. The ALD-FN primer is an unlabeled polynucleotide. The labeling of the polynucleotide with the fluorescent substance can be carried out in the conventional manner.

The ALD-R primer hybridizes with a part of the aldolase gene region, which is the target sequence. It is used as a reverse primer in the PCR amplification reaction.

The forward primer solution for the PCR amplification reaction was prepared by mixing the ALD-FF primer and ALD-FN primer in a concentration ratio of 1:100 to give a total concentration of 25 µM.

The reverse primer solution was prepared at an ALD-R primer concentration of 25 µM and used.

### [Amplification reaction step]

This step is described referring to Fig. 4. In this step, the PCR amplification reaction is carried out using the primers mentioned above.

The template genomic DNA (1 µg), 5 µL of 2 mM dNTPs, 1.5 µL of 50 mM MgCl₂, 5 µL of 10 x Tsp buffer, 35.1 µL of sterilized water, 0.4 µL of Tsp DNA polymerase, 1 µL of the 25 µM forward primer mixture solution (mixture solution with the ratio ALD-FF primer:ALD-FN primer = 1 : 100), and 1 µL of the 25 µM reverse primer solution are mixed up. As for the PCR conditions, the initial denaturation is carried out at 94°C for 2 minutes, the amplification temperature cycle comprising 30 seconds at 94°C, 30 seconds at 57°C and 60 seconds at 72°C is repeated 10 times, then the cycle comprising 30 seconds at 89°C, 30 seconds at 57°C and 60 seconds at 72°C is repeated 20 times and, finally, the extension reaction is carried out at 72°C for 7 minutes. The PCR-amplified product nucleic acid is then detected. This is to be described later herein.

For comparison, the PCR reaction was carried out in the conventional manner. In this case, an ALD-FF primer solution alone was used as the forward primer solution. The reagent concentrations, liquid volumes, reaction conditions and other conditions were the same as mentioned above. The yield of DNA formed under the PCR conditions in this example according to the invention and the yield of DNA formed under the conventional PCR conditions were compared. For the comparison, the well-known technique of gel electrophoresis is used. The apparatus used for gel electrophoresis was Mupid (product of Advance Co.). The gel used was agarose. First, 1 µL of 6 x sample buffer was added to 5 µL each of the reaction mixture obtained in this example and the conventional PCR reaction mixture, and the mixtures were poured into different injection ports of the gel in Mupid. Then, electrophoresis was carried out at a constant voltage of 100 V for 50 minutes. Thereafter, the whole gel was stained with a 5 mg/L ethidium bromide solution. When there is a double-stranded DNA, ethidium bromide is incorporated between the strands and emits strong fluorescence upon ultraviolet irradiation. The amount of DNA can be determined by measuring the intensity of this fluorescence. Upon electrophoresis, the template DNA and the DNA produced by the PCR are separated as bands (based on the difference in molecular weight). As a result of comparison between the fluorescence intensities of the bands of the product DNA, it was found that the yield of DNA formed under the PCR conditions in this example was almost the same as the yield of DNA formed under the conventional PCR conditions. It could be confirmed that the PCR using the forward primer mixture solution in this example does not differ from the conventional PCR; no difference in PCR reaction was observed. Thus, the PCR amplification reaction can be carried out under the same conditions as the ordinary conditions even when the forward primer mixture solution prepared in this example is used. In the above-mentioned detection of the ethidium bromide-due fluorescence upon ultraviolet irradiation, the influence of the fluorescent substance FAM had been eliminated; hence, there is no influence of the fluorescent substance FAM on the DNA quantitation.

In addition to the above example, forward primer mixture solutions were prepared by varying the ALD-FF primer-to-ALD FN primer concentration ratio from 1:1 to 1:10,000 and evaluated for utility in the PCR amplification reaction. As a result of confirmation of the yields of DNA by the same gel electrophoresis as in the above example, it was found that the yield of DNA formed by the PCR was almost the same in each of the cases examined. This indicates that the primer mixture solutions in this example hardly inhibit the amplification reaction itself. Thus, when the primers of this example are used, the conventional conditions can be employed as such, and it is therefore easy to shift from the conventional PCR amplification reaction schemes. The primers are also applicable to other methods of amplification.

### [Detection step]

In this step, the product DNA prepared in the step mentioned above is detected. While various methods and apparatus can be applied for the detection, the case of SSCP analysis using an apparatus for capillary electrophoresis is described in this example.

The apparatus to be used for capillary electrophoresis is Applied Biosystems' ABI PRISM™ 3100 Genetic Analyzer. A capillary with an inside diameter of 75 µm and a detection length of 36 cm as produced by the inventors is used. It is packed with Applied Biosystems' GeneScan polymer concentrated by the inventors and used for electrophoresis and detection.

A 3-µL portion of the reaction mixture containing the PCR product DNA prepared in the above amplification reaction step using the forward primer mixture solution is mixed with 37 µL of formamide containing an adequate amount of a well-known labeled fragment marker (oligomer having a known base length), and thermal denaturation is carried out at 94°C for 2 minutes. Then, the mixture is cooled on ice and used as the sample solution for capillary electrophoresis. The reaction mixture containing the product DNA is used as such without dilution. As for the electrophoresis conditions, the capillary temperature is controlled at 18°C, sample injection is performed at 12 kV over 5 seconds, and electrophoretic separation is effected at 12 kV for 50 minutes. As for the signal intensity to be analyzed, the baseline and amplitude are varied by the inventors and used based on the data obtained by electrophoresis of standards.

Fig. 6 shows, by way of example, some results of electrophoresis as obtained in this example. The abscissa denotes the duration of electrophoresis, and the ordinate denotes the fluorescence intensity as detected. The peak at the position corresponding to 33 minutes is the peak of the fragment marker, and the peaks appearing at about 39 minutes and about 41 minutes of electrophoresis are the peaks of the PCR-amplified product DNAs. Since the template is the genomic DNA and the target nucleic acid is amplified from both of the father-derived and mother-derived genomes, the products appear as two peaks. By analyzing these two peaks, it is possible to carry out diagnosis, for instance. In addition, it is also possible to obtain the information about the presence or absence of a gene or about polymorphism, for instance, by measuring and analyzing the peaks obtained by electrophoresis of various samples.

Fig. 5 shows the results of electrophoresis as obtained with a forward primer mixture solution at an ALD-FF primer-to-ALD-FN primer concentration ratio of 1:50. The signal peaks were obtained at the same positions as in Fig. 6. The area or integrated intensities of the product DNA peaks were about twice higher as compared with those in Fig. 6.

For comparison, 3 µL of the reaction mixture obtained under the conventional conditions was electrophoresed and measured by the same procedure as mentioned above. The fluorescence intensities of the product DNAs of interest were too strong and overflowed the signal intensity limit of the detector, resulting in failure of exact measurements. A 3-µL portion of the reaction mixture sample was diluted by adding 297 µL of a diluent, and 3 µL of this dilution was taken out with a pipette and subjected to electrophoresis by the same procedure as mentioned above, whereupon almost the same results of electrophoresis were obtained and the same results of analysis were obtained.

As mentioned above referring to the amplification reaction step, the use of forward primer mixture solutions varying in ALD-FF primer-to-ALD-FN primer concentration ratio from 1:1 to 1:10,000 gave almost the same DNA yields (also the same as in the conventional method). The reaction mixtures obtained by using forward primer mixture solutions varying in mixing ratio were subjected to electrophoresis; the sizes of the electrophoretic peaks increased or decreased depending on the mixing ratio. In particular when the ALD-FF primer-to-ALD-FN primer concentration ratio was in the range from 1:10 to 1:500, it could be confirmed that the concentration ratio is almost proportional to the peak area intensities. Therefore, preferably, the ALD-FF primer-to-ALD-FN primer concentration ratio is adjusted within the range of 1:1 to 1:10,000, and a forward primer mixture solution that will give appropriate intensity levels is selected and used depending on the sensitivity of the detector in each instance. In that case, the detection step can be begun without carrying out any dilution step, so that the system can be simplified and the apparatus becomes simple to construct and easy to handle.

Furthermore, labeled primers are more expensive as compared with unlabeled primers. In this example, it is possible to reduce the amount of the labeled primer (in the instant case, the ALD-FF primer) depending on the sensitivity of the detection apparatus, so that it becomes possible to reduce the overall reagent cost.

According to this example, the detection step can be carried out directly without diluting the reaction mixture after the amplification reaction by PCR or the like. Therefore, the operations become easier and, in the case of constructing an automated apparatus, the system can be simplified and the apparatus becomes more simplified and less expensive.

Furthermore, the amount of the labeled primer, which is expensive, can be reduced depending on the sensitivity of the detection apparatus. Therefore, the overall reagent cost can be cut down and the running cost can be reduced.

While the fluorescent substance FAM was used as the label in the above example, the label is not limited thereto. Thus, various fluorescent substances and, further, chemiluminescent substances, bioluminescent substances, phosphorescent substances, radioisotopes, fine particles and various other labels can be used to give the same effects as mentioned above.

Further, it is also possible to use, as the modifying group, the phosphoric acid group, amino group, thiol group, biotin, digoxigenin and the like. A fluorescent substance or chemiluminescent substance can easily be coupled to any of such modifying groups.

The method of amplification is not limited to the PCR employed in this example but various methods can be employed to give the same effects as mentioned above. Thus, the RCR (repair chain reaction), TAS, 3SR, NASBA, SPSR, LAT and SDA methods, among others, may be employed without any particular restriction provided that the above-defined oligomer mixture is used therein.

In cases where a reaction other than the amplification of the target nucleic acid is simultaneously carried out (e.g. in RT-PCR), too, a labeled primer and the corresponding unlabeled primer are mixed together in a mixing ratio of 1:1 to 1:10,000, more preferably 1:10 to 1:500, and the reaction is carried out using the mixture in a total amount adequate for the reaction. This makes the luminescence intensity or radioactivity in the product nucleic acid appropriate for detection or analysis.

Since the ratio between the ALD-FF primer and ALD-FN primer in the primer mixture solution is almost proportional to the integrated intensity of the peaks separated, it is also possible to calculate the total amount of the product nucleic acids by dividing the integrated intensity by the ALD-FF primer-to-ALD-FN primer ratio.

As for the method of measuring the label in the product DNA after the amplification reaction, not only the method mentioned above but also apparatuses in which detection is carried out while separating the product nucleic acid(s), for example a detecting apparatus in which gel electrophoresis using a plate gel is employed, can be used. Further, when a homogeneous detection method using the well-known fluorescence polarization measurement or time-resolved phosphorescence detection energy resonance, for instance, is used, a fluorophotometer, a microplate reader or the like can also be used. Alternately, the step of removing the unreacted oligomers may be carried out.

### Example 2

A nucleic acid treatment apparatus (system) based on the method of the invention which uses a labeled/unlabeled primer mixture is now described.

The main apparatus constitution comprises an extraction section, amplification section, and detection section, as shown in Fig. 2.

The extraction section comprises a mechanism for extracting the template nucleic acid containing the target nucleic acid from a sample. This is an automated unit having the same function as that of the step of template DNA extraction from a sample as described in Example 1. For example, it comprises the mechanism section, control section and other sections which have the constitution disclosed in JP-A 2000-166556.

The amplification section comprises a mechanism for amplifying the target nucleic acid using the forward primer mixture solution described in Example 1, for instance. It stores the reagent solutions described in Example 1 for use in the amplification reaction step, including the forward primer mixture solution, and is constituted of a temperature cycler for amplification, reagent/solution sucking up and distributing nozzles like those in the mechanism section of the extraction section, tips for distribution, holders for tips for distribution, reaction vessels, tip extractors, arms for transfer in the xyz directions, and so forth. It is also possible to utilize the unit of the above-mentioned extraction section.

The detection section comprises a mechanism for detecting and analyzing the product nucleic acid(s).

In the following, a typical embodiment is described referring to the drawings attached hereto. In this embodiment, the apparatus is constituted of two divided units, namely a unit including the extraction section and amplification section and a unit for detection, although the apparatus may be constituted by uniting both units.

The unit including the extraction section and amplification section is described. Fig. 7 is an external view of the extraction and amplification section 100, Fig. 8 is a plan view of the extraction and amplification section 100, and Fig. 9 is a drawing illustrating how to attach each tip to a nozzle.

The details of the basic actions or movements are as described in JP-A 2000-166556.

Syringes 10 and 32 each controls the liquid sucking up and discharging. The syringes 10 and 32 are respectively and independently connected to nozzles 36 and 39 via a piping and a nozzle holder 17 and via a piping and a nozzle holder 34, respectively. The distribution nozzle 36 and movable nozzle 39 for liquid sucking up and discharging are fixed on the nozzle holders 17 and 34, respectively, and the nozzle holders 17 and 34 are fixed on respective arms 16 and 33 in a state such that they can move in the Y direction (longitudinal direction of the arms 16 and 33 shown in Fig. 7) and in the Z direction (direction perpendicular to the longitudinal direction of the arms 16 and 33 shown in Fig. 7).

The arms 16 and 33 can move independently in the X direction (direction perpendicular to the above-mentioned Y direction and to the above-mentioned Z direction) and, when given a difference in position in the Z direction, they can overlap with each other in the X direction. This makes it possible for the nozzles to move to necessary planar sites on the apparatus by combinations of the movements of the nozzle holders 17 and 34 and the arms 16 and 33.

The movements of these syringes, arms, nozzle holders and so forth are controlled by a control operation section.

Each tip holder 14 (Fig. 7 and Fig. 8) for distribution can hold distribution tips 15, and a plurality of the same tip holders 14 (in the figures, 4 holders 14a, 14b, 14c and 14d) can be disposed on the apparatus. Forty-eight reaction vessels 24 can be disposed on a reaction vessel rack 23, and forty-eight purified product containers 26 can be disposed on a purified product rack 25. A cold insulation mechanism (not shown) is disposed under the purified product rack 25 and can keep the purified product rack 25 cold.

On the apparatus, there can be disposed one washing solution bottle 19, one eluent bottle 20, one diluent bottle 21, one binding promoter bottle 22, and one container 200 containing reagents for the amplification reaction. On a nucleic acid trapping tip rack 30, there can be disposed 48 distribution tips 31. At the bottom of each of the nucleic acid colleting tip rack 30 and binding promoter bottle 22, there is disposed a warming mechanism (not shown) and can warm each bottle. The reagent container 200 for the amplification reaction is contained in and cooled by a cooling unit 201.

On the apparatus, there is disposed a temperature cycler 202 for the amplification reaction.

On the occasion of tip attachment, the movements of the arm 16 and nozzle holder 17 are controlled so that the nozzle 36 may move to a site just above the desired distribution tip 15 on the tip holder 14. Then, the nozzle holder 17 is moved downward so that the nozzle 36 may come into contact with the specified site of the distribution tip 15. This can result in automatic attachment of the distribution tip 15 to the tip of the nozzle 36. By controlling the nozzle 39, nozzle holder 34 and arm 33 in the same manner, it is possible to attach the nucleic acid trapping tip 31 to the tip of the nozzle 39.

Tip detachment is realized by using a tip extractor 27 while controlling the movements of the arm 16 and nozzle holder 17. The nozzle holder 34 and arm 33 are also operated in the same manner.

Liquid receptacles 11 and 28 can receive the discharge liquids from the nozzles 36 and 39, and the liquids received are sent as waste liquids. A washing section 18 can wash the distribution tip attached to the nozzle holder 17 via the nozzle 36 by discharging running water.

As the nucleic acid trapping tip 31, use is made of the one described in JP-A 2000-166556. The movements for collecting, recovering and amplifying the sample nucleic acid having the target nucleic acid in this case are now described.

First, the distribution tip 15 is attached to the nozzle 36 by the predetermined movements while controlling the movements of the arm 16 and nozzle holder 17. Then, a predetermined amount of the binding promoter is sucked up from the binding promoter bottle 22 and further a predetermined amount of air is sucked up by controlling the movements of the arm 16 and nozzle holder 17 and the syringe 10. The distribution tip 15 is moved to a washing table 18, and the outer wall of the distribution tip 15 is washed with running water.

After washing of the outer wall of the distribution tip 15, the nozzle holder 17 is moved to the site of a predetermined sample 13 on a sample rack 12. The movement of the syringe 10 is controlled and a predetermined amount of the sample is sucked up into the distribution tip 15. After sucking up of the sample into the distribution tip 15, the nozzle holder 17 is moved to a site above the reaction vessel rack 23, and the whole amount of the sample in the distribution tip 15 is discharged into a predetermined reaction vessel 24 on the rack 23.

After discharge of the sample into the reaction vessel 24, the sample in the reaction vessel 24 is further sucked up and discharged by the distribution tip 15 to thereby mix up the sample and the binding promoter. After mixing up of the sample and binding promoter, the nozzle holder 17 is moved to the site of the tip extractor 27, and the distribution tip 15 is detached from the nozzle 36 by the predetermined operation mentioned above.

The nucleic acid trapping tip 31 is attached to the nozzle 39 by the predetermined operation by controlling the movements of the arm 33 and nozzle holder 34. Then, the nozzle holder 34 is moved to the reaction vessel 24 containing the above-mentioned mixed solution (mixed solution containing the sample and binding promoter) on the reaction vessel rack 23, and the above mixed solution is sucked up into the inside of the nucleic acid trapping tip 31 by controlling the movement of the syringe 32.

After repetition of a predetermined number of times of aspiration and discharging, the mixed solution in the reaction vessel 24 is sucked up into the nucleic acid trapping tip 31 and the tip 31 is then moved to a waste liquid port 29 by controlling the movements of the arm 33 and nozzle holder 34. The mixed solution in the nucleic acid trapping tip 31 is discharged into the waste liquid port 29. After discharging of the mixed solution into the waste liquid port 29, the tip 31 is moved to a liquid receptacle 28 by controlling the movements of the arm 33 and nozzle holder 34.

A distribution tip 15 is attached to the nozzle 36 by controlling the movements of the arm 16 and nozzle holder 17 and, then, a predetermined amount of a washing solution is sucked up from the washing solution bottle 19 by controlling the movements of the arm 16 and nozzle holder 17 and the syringe 10. The nozzle holder 17 is then operated to cause the washing solution to be discharged into the predetermined reaction vessel 24 on the reaction vessel rack 23.

After discharging of the washing solution, the nozzle holder 17 is moved to the site of the tip extractor 27 by controlling the movements of the arm 16 and nozzle holder 17, and the distribution tip 15 is removed from the nozzle 36 by the predetermined operation.

After the movement of the nozzle holder 17, the nucleic acid trapping tip 31 is moved to the washing solution-containing predetermined reaction vessel 24 on the reaction vessel rack 23 by controlling the movements of the arm 33 and nozzle holder 17. The washing solution is sucked up into the nucleic acid trapping tip 31 through the movement of the syringe 32. After sucking up of the washing solution, sucking up and discharging are repeated a predetermined number of times by operating the syringe 32 for washing the solid phase 38 with the washing solution. After repetition of the predetermined number of times of sucking up and discharging, the washing solution in the reaction vessel 24 is sucked up into the nucleic acid trapping tip 31. Then, the nucleic acid trapping tip 31 is moved to the waste liquid port 29 by controlling the movements of the arm 33 and nozzle holder 34, and the washing solution in the nucleic acid trapping tip 31 is discharged by operating the syringe 32. After discharging of the washing solution, the nucleic acid trapping tip 31 is moved to the liquid receptacle 28 by operating the arm 33 and syringe 32.

The above nucleic acid washing step is repeated a predetermined number of times according to need. Although the above-mentioned nucleic acid washing step as such may be repeated on that occasion, the nucleic acid washing step can be repeated efficiently by sucking up the washing solution in an amount sufficient for a plurality of times of washing into the distribution tip 15 and discharging a necessary amount thereof into the reaction vessel 24 and, after discharging of the washing solution, moving the tip 15 to the site of the liquid receptacle 11 and, after washing operation for the nucleic acid trapping tip 31, again causing a necessary amount of the washing solution to be discharged into the reaction vessel 24 from the distribution tip 15.

Then, after attaching a distribution tip 15 to the nozzle 36 by controlling the movements of the arm 16 and nozzle holder 17, a predetermined amount of the eluent is sucked up from the eluent bottle 20 by operating the arm 16 and nozzle holder 17 and the syringe 10, and the eluent is discharged into the predetermined reaction vessel 24 on the reaction vessel rack 23 by operating the nozzle holder 17.

After discharging of the eluent, the nozzle holder 17 is moved to the site of the tip extractor 27 by controlling the movements of the arm 16 and nozzle holder 17, and the distribution tip 15 is removed from the nozzle 36 by the predetermined operation.

After the movement of the nozzle holder 17, the nucleic acid trapping tip 31 is moved to the eluent-containing predetermined reaction vessel 24 on the reaction vessel rack 23 by controlling the movements of the arm 33 and nozzle holder 34. And, the eluent is sucked up into the nucleic acid trapping tip 31 by operating the syringe 32. After sucking up of the eluent, sucking up and discharging are repeated a predetermined number of times by operating the syringe 32 to thereby bring the solid phase 38 into contact with the eluent.

Eluent sucking up and discharging are repeated a predetermined number of times and the eluate in the reaction vessel 24 is sucked up into the nucleic acid trapping tip 31. Then, the nucleic acid trapping tip 31 is moved to a predetermined purified product receptacle 26 contained in the purified product rack 25 by controlling the movements of the arm 33 and nozzle holder 34. The eluate in the nucleic acid trapping tip 31 is discharged into the receptacle 26 by operating the syringe 32. After eluate discharging, the nucleic acid trapping tip 31 is moved to the liquid receptacle 28 by operating the arm 33 and nozzle holder 34. The above elution step is repeated a predetermined number of times according to need. Although the above elution step as such may be repeated on that occasion, the above elution step can be repeated efficiently by sucking up the eluent in an amount sufficient for a plurality of elution operations into the distribution tip 15, discharging a necessary amount thereof into the reaction vessel 24 and, after eluent discharging, moving the distribution tip 15 to the site of the liquid receptacle 11 and, after the sucking up and discharging operation of the nucleic acid trapping tip 31, again causing a necessary amount of the eluent to be discharged into the reaction vessel 24 from the distribution tip 15.

After completion of the above step, the nozzle holder 34 is moved to the site of the tip extractor 27 by controlling the movements of the arm 33 and nozzle holder 34, and the nucleic acid trapping tip 31 is dismounted from the nozzle 39 by the predetermined operation. The arm and nozzle holder then move to the right corner.

In the above step, a genomic DNA solution can be obtained from the sample.

Then, the amplification reaction is carried out.

The reagent container 200 for the amplification reaction contains such a premixed reagent solution for the amplification reaction as described in Example 1 (a premix solution comprising dNTPs, MgCl₂, Tsp buffer, sterilized water, Tsp DNA polymerase, forward primer mixture solution, reverse primer solution and so forth).

The procedure for using the genomic DNA solution in the purified product receptacle 26 and amplifying the target nucleic acid contained therein is now described. A distribution tip 15 on the tip holder 14d is attached to the nozzle 36 by controlling the movements of the arm 16 and nozzle holder 17. Then, a predetermined amount of the premixed reagent solution for the amplification reaction is sucked up into the distribution tip 15 from the reagent container 200 for the amplification reaction by controlling the movements of the arm 16 and nozzle holder 17 and the syringe 10. Then, the solution in the distribution tip 15 is discharged into a predetermined amplification vessel 203. Further, a necessary amount of the genomic DNA solution is transferred to the predetermined amplification vessel 203 from the purified product receptacle 26, followed by mixing up. Thereafter, such an amplification operation as PCR is carried out under predetermined conditions. The descriptions of the opening and closing operations of the cover of the amplification vessel 203 and the cover of the temperature cycler are omitted.

In the above step, the target nucleic acid is amplified.

Then, formamide is mixed with the solution (a part) after the amplification reaction and, after 2 minutes of thermal denaturation at 94°C, the mixture is subjected to measurement on a well-known detection apparatus, for example an apparatus for capillary array electrophoresis.

While a premixed reagent solution for the amplification reaction was used in the step of amplification in this example, the respective reagents may be stored separately, for instance. The apparatus may be constituted in a manner such that the labeled primer and unlabeled primer are stored separately and, after obtaining the relevant information from the detection apparatus, the mixing ratio is determined prior to amplification, they are then mixed up and the amplification reaction is carried out. In that case, a necessary number of container storage sites are prepared within the apparatus unit and the necessary distribution operations are carried out.

In this example, the operation of the dilution step can be omitted, so that the apparatus constitution becomes simplified.

### EFFECTS OF THE INVENTION

The amount of the labeled or modified oligomer can be reduced in nucleic acid amplification. Therefore, the cost of nucleic acid amplification treatment and nucleic acid analysis treatment can be reduced and/or the process can be simplified.

## Claims

1. A method of nucleic acid amplification by using at least a mixture of the oligomers defined below, a nucleic acid containing a target nucleic acid, and reagents for amplification to produce a product nucleic acid comprising a sequence related to said target nucleic acid:
A luminescent substance- or radioisotope-labeled or modifying group-coupled oligomer A capable of hybridizing with an arbitrary specific sequence contained in the target sequence; and
An oligomer B neither labeled with the same substance as in the oligomer A nor coupled with the modifying group coupled to the oligomer A but capable of hybridizing with said specific sequence.

2. A nucleic acid amplification method as set forth in Claim 1, wherein the mixing ratio of said oligomer A to said oligomer B is 1:1 to 1:10,000.

3. A nucleic acid amplification method as set forth in Claim 2, wherein the mixing ratio of said oligomer A to said oligomer B is 1 :10 to 1 :500.

4. A nucleic acid amplification method as set forth in Claim 1, wherein the luminescent substance is a fluorescent substance or a chemiluminescent substance.

5. A nucleic acid amplification method as set forth in Claim 1, wherein the modifying group-coupled oligomer A is a biotinylated, phosphorylated, aminated, digoxigenin-coupled, or thiolated oligomer.

6. An oligomer kit comprising the oligomers defined below and capable of producing a product nucleic acid comprising a sequence related to a target nucleic acid:
A luminescent substance- or radioisotope-labeled or modifying group-coupled oligomer A capable of hybridizing with an arbitrary specific sequence contained in the target sequence; and
An oligomer B neither labeled with said luminescent substance or radioisotope labeling the oligomer A nor coupled with the modifying group coupled to the oligomer A but capable of hybridizing with said specific sequence.

7. An oligomer kit as set forth in Claim 6, wherein the abundance ratio of said oligomer A to said oligomer B is 1:1 to 1:10,000.

8. An oligomer kit as set forth in Claim 7, wherein the abundance ratio of said oligomer A to said oligomer B is 1:10 to 1:500.

9. An oligomer kit as set forth in Claim 6, wherein the luminescent substance is a fluorescent substance or a chemiluminescent substance.

10. An oligomer kit as set forth in Claim 6, wherein the modifying group-coupled oligomer A is a biotinylated, phosphorylated, aminated, digoxigenin-coupled, or thiolated oligomer.

11. A method of analyzing nucleic acids which comprises the following steps:
The step of amplification in which at least a mixture of the oligomers defined below, a nucleic acid containing a target nucleic acid and reagents for amplification are used to produce a product nucleic acid comprising a sequence related to said target nucleic acid;
(A) A luminescent substance- or radioisotope-labeled or modifying group-coupled oligomer A capable of hybridizing with an arbitrary specific sequence contained in the target sequence; and
(B) An oligomer B neither labeled with said luminescent substance or radioisotope labeling the oligomer A nor coupled with the modifying group coupled to the oligomer A but capable of hybridizing with said specific sequence;
The step of coupling in which a luminescent substance is coupled to said modifying group when said oligomer A is a modifying group-coupled one; and
The step of detecting said luminescent substance or radioisotope.

12. An analytical method as set forth in Claim 11 which further comprises the following step:
The step of operation in which the amount of the product nucleic acid is calculated based on the amount of the luminescent substance or radioisotope detected.

13. An analytical method as set forth in Claim 12, wherein, in said operation step, the amount of the product nucleic acid is calculated based on the mixing ratio between said oligomer A and said oligomer B.

14. An analytical method as set forth in Claim 11, wherein the concentration of the luminescent substance- or radioisotope-labeled target nucleic acid in the product nucleic acid-containing reaction mixture at the time of completion of said amplification step or at the time of completion of said coupling step is within the range within which said concentration can be measured by the detection apparatus used in said detection step.

15. An apparatus for producing a product nucleic acid comprising a sequence related to a target nucleic acid which apparatus comprises the following constituent elements:
A holding vessel A for holding a luminescent substance- or radioisotope-labeled or modifying group-coupled oligomer A capable of hybridizing an arbitrary specific sequence contained in said target nucleic acid;
A holding vessel B for holding an oligomer B neither labeled with said luminescent substance or radioisotope labeling the oligomer A nor coupled with the modifying group coupled to the oligomer A but capable of hybridizing with said specific sequence;
A nucleic acid amplification vessel capable of holding an aqueous solution containing at least said oligomer A, said oligomer B, reagents for amplification, and a nucleic acid containing said target nucleic acid; and
A feeding mechanism for feeding a predetermined amount of said oligomer A and a predetermined amount of said oligomer B to said nucleic acid amplification vessel.

16. A production apparatus as set forth in Claim 15, wherein the feeding ratio between said oligomer A and said oligomer B to be fed to said feeding mechanism is 1:1 to 1:10,000.

17. A production apparatus as set forth in Claim 16, wherein the feeding ratio between said oligomer A and said oligomer B to be fed to said feeding mechanism is 1:10 to 1:500.

18. A nucleic acid analyzing apparatus for detecting a target nucleic acid which comprises the following constituent elements:
A nucleic acid amplification vessel capable of holding the oligomers defined below, reagents for amplification and a nucleic acid containing said target nucleic acid and capable of producing a product nucleic acid comprising a sequence related to said target nucleic acid;
(A) A luminescent substance- or radioisotope-labeled or modifying group-coupled oligomer A capable of hybridizing with an arbitrary specific sequence contained in the target sequence; and
(B) An oligomer B neither labeled with said luminescent substance or radioisotope labeling the oligomer A nor coupled with the modifying group coupled to the oligomer A but capable of hybridizing with said specific sequence; and
a detection mechanism for detecting said luminescent substance or radioisotope or a luminescent substance coupled to said modifying group.

19. A nucleic acid analyzing apparatus as set forth in Claim 18 which further comprises the following constituent element:
A display mechanism for displaying the information depending on the amount of said product nucleic acid as calculated based on the amount of the luminescent substance detected.

20. A nucleic acid analyzing apparatus as set forth in Claim 19, wherein said display mechanism calculates the amount of said product nucleic acid based on the mixing ratio between said oligomer A and said oligomer B.
